# EUROPEAN PATENT SPECIFICATION

(11) **EP 1 581 126 B1**
(45) Date of publication and mention of the grant of the patent: **19.08.2009**
(21) Application number: 03779761.0
(22) Date of filing: 18.12.2003
(51) Int. Cl.: A61B 17/50

(54) **TICK REMOVER**
ZECKENENTFERNER
INSTRUMENT POUR L'ELIMINATION DE TIQUES

(30) Priority: 18.12.2002 DK 200201931
(43) Date of publication of application: 05.10.2005
(73) Proprietor: Glaesel, Jörgen, 2900 Hellerup (DK)
(72) Inventor: Glaesel, Jörgen, 2900 Hellerup (DK)
(74) Representative: Noergaard, Tage
(86) International application number: PCT/DK2003/000905
(87) International publication number: WO 2004/054457

(56) References cited:
- US-A- 5 116 347
- US-A- 5 595 569
- US-A- 5 607 434
- US-A- 5 876 409
- US-A- 6 102 919

## Description

The invention relates to a tool for removing ticks and the like parasites from the skin, which tool has an engagement part with a bottom face and a top face, wherein a V-shaped groove is provided that has side faces between the bottom face and the top face at an edge of the engagement part, which side faces converge towards each other from an outer opening at the edge towards an internal assembly point, said V-shaped groove being wider at the top face than at the bottom face, a considerable portion of each side face of the V-shaped groove between the bottom face and the top face of the engagement part being constituted by a concave engagamenet face, the lowermost part of the concave engagement face being substantially parallel with the bottom face, the concave shape of the concave engagement face being formed by a continously curved line segment.

From the specification of US patent No 5116347 a tool for moving ticks and the like parasites from the skin is known; said tool has an engagement part with a bottom face and a top face wherein a V-shaped indentation is provided that has side faces between the bottom face and the top face at an edge of the engagement part; the side surfaces converge towards each other from an outer opening at the edge towards an internal assembly joint; the V-shaped indentation is wider at the top face than at the bottom face, and a considerable portion of each side face of the V-shaped indentation between the bottom face and the top face of the engagement part is constituted by a concave engagement face. This means that during removal of a tick the head and optionally the body of the tick is wedged firmly between the two side faces and the removal as such of the tick does not occur until the user subsequently displaces and lifts the tool. This manipulation of the tick prior to the concluding removal involves a risk that the tick has time to transmit pathogenic bacteria to the host before the tick is completely removed.

It is therefore the object of the invention to provide a tool by which it is possible to remove ticks (and other like parasites) in a simple and efficient manner, while minimising the risk of transmission of pathogenic bacteria to the host.

According to the invention the tool is characterized in that at least for a distance the lowermost part of the side face is increasing in height, seen from the edge to the internal assembly point. As a result a tool is obtained by which, by one single sliding movement of the tool, the tick may be lifted, while simultaneously it is wedged between the lowermost parts of the side faces of the V-shaped groove. By lifting the tick the risk of the tick transmitting pathogenic bacteria is clearly minimised.

According to another preferred embodiment of the invention the concave shape of the engagement face may be formed of a continuously curved line segment that may have the shape of a drawing of a circle, or it may be formed of a plurality of straight lines. Irrespective of how the concave shape is generated, a space is formed between the side faces for the head and/or the body of the tick.

According to another preferred embodiment, each side face comprises a lowermost part that extends substantially perpendicularly upwards from the bottom side and is connected to the concave engagement face. By configuring the V-shaped groove in this manner requisite rigidity and strength of the side faces of the V-shaped groove is obtained, thereby ensuring that they do not unintentionally flex outwards. Furthermore the risk that the lowermost edge of the side faces cuts the skin when the tool is used for removing a tick is reduced.

Preferably the lowermost part of the side face has a constant low height a distance from the edge into the V-shaped indentation. Hereby it is ensured that even small ticks are not wedged firmly between the side faces, but are rather seized from below by the engagement faces and lifted off the skin.

At the bottom of the V-shaped groove a cutter blade may advantageously be provided in level with the bottom face, whereby a tick that cannot be removed by the tool is at least cut off by a clean cut.

According to a preferred embodiment the tool may comprise a holder part provided with means for ensuring an ergonomically correct grip, which means may comprise an upwardly protruding transversal beam for supporting a thumb, an indentation for supporting a thumb, or a ribbed area for supporting a thumb, and/or at least one recess at the edge for supporting at least one finger.

Further according to the invention the tool may be plate-shaped, whereby, on the one hand, it is easy to store the tool and, on the other, it is easy to impart thereto a desired flexibility for obtaining adequate and close contact with the skin in use.

In order to impart further flexibility to the tool, it may be an option to provide at least one recess in an area between the engagement part and the holder part.

Finally according to the invention, the largest outer dimension of the tool may correspondP essentially to the size of a credit card. Hereby the tool is readily kept along with credit cards and other cards of same standard dimension.

The invention will now be explained in further detail with reference to the drawing, wherein
Figure 1 shows a tool according to the invention in a first embodiment,
Figure 2 shows an enlarged view of the engagement part of the tool shown in Figure 1,
Figure 3 shows a cross sectional view of the side face of the V-shaped part of the engagement part shown in Figure 2,
Figure 4 shows a longditudinal-sectional view through the engagement part shown in Figure 2 during removal of a tick,
Figure 5 shows how the tool shown in Figure 1 is used to remove a tick,
Figure 6 shows a tool according to the invention in a second embodiment,
Figure 7 shows a tool according to the invention in a third embodiment,
Figure 8 shows an alternative embodiment of the engagement part of a tool according to the invention, and
Figure 9 shows a cross sectional view of the side face of the V-shaped groove in the engagement part shown in Figure 8.

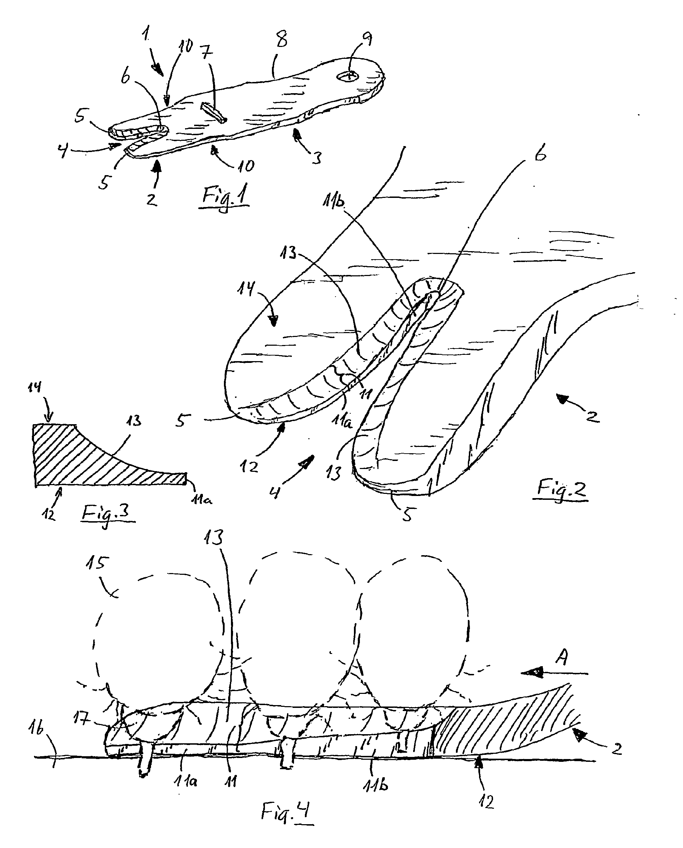

Figure 1 shows a first embodiment of the tool 1 for removing ticks and the like parasites. The tool 1 comprises an engagement part 2 and a holder part 3. The engagement part 2 is provided with means for removing a tick from the skin of a person, and the holder part 3 is provided with means for accomplishing optimal seizing of the tool 1 during use.

The engagement part 2 is provided with a V-shaped indentation 4 that extends from an edge 5 of the engagement part 2 to an assembly point 6; the inventive tool is configured as shown more detailed in Figures 2-4, which will be subject to a more detailed description later in this description.

In the embodiment shown in Figure 1, the holder part 3 comprises an upwardly protruding transverse beam 7 that serves as stop for the user's thumb, and a recess 8, the edge of which serves as a stop for the user's index finger when the tool 1 is used for removing a tick. The holder part 3 may further be provided with an aperture 9, enabling the tool 1 to be carried in a chain or string that extends through the aperture 9, or it can be inserted into a key ring.

As will appear - conf. Figure 1 - the engagement part 2 is narrower than the holder part 3, since auxiliary recesses 10 are provided that make the engagement part 2 of the tool 1 more flexible than the holder part 3. This is of consequence to the functioning of the tool 1, as will appear below.

The tool 1 is preferably made of a comparatively hard, yet flexible plastics material; however, it may also be manufactured from eg metal.

Figure 2 shows the engagement part 2 of the tool 1 shown in Figure 1 in an enlarged scale. As it appears, the V-shaped indentation 4 extends inwardly from the edge 5 of the engagement part 2. The edge 5 is rounded to minimise the risk of damaging the skin when the tool 1 is used, but also to minimise the risk of harming or destroying the tick during use of the tool 1.

The V-shaped indentation 4 is configured with opposing side faces 11 that extend from a bottom side 12 of the engagement part 2 to its top side 14. A lowermost part 11 a of a side face 11 extends substantially perpendicularly upwards from the bottom face 12 of the engagement part 2. Each side face 11 further comprises an engagement face 13 that extends from the lowermost part11 a of the side face 11 to the top side 14 of the engagement part 2. As it appears the transition between the lowermost part 11a of the side face 11 and the engagement face 13 extends in the outer part of the V-shaped indentation 4, substantially in parallel with the bottom face 12 of the engagement part 2, while - further into the V-shaped indentation 4 - it diverges from the bottom face 12. This means that the distance between the engagement face 13 and the bottom face 12 increases the closer the engagement face 13 is to the assembly point 6.

The V-shaped indentation 4 may be provided with straight side faces seen from above; preferably, however, it is provided with curved side faces as shown in Figure 2. The advantage of using curved side faces instead of straight side faces is that it is hereby possible to configure the engagement part 2 in such a way that the most careful removal of ticks is ensured. Hereby the risk is reduced of the tick having the time to transmit pathogenic bacteria prior to being completely removed.

The side face 11 comprises a concavely configured engagement face 13, as will appear in a perspective view in Figure 2, and yet more clearly from the sectional view shown in Figure 3. When said engagement face 13 is made to be concave, a hollow is provided that provides ample space for receiving the head and/or body of a tick without it being squeezed between the opposing engagement faces 13 of the V-shaped indentation 4. The concave shape is preferably configured like the drawing of a circle; yet other configurations, however, are possible, see the below teachings.

In the embodiment shown in Figure 2 the innermost segment of the V-shaped indentation 4 is provided with parallel side faces11b and with a rounded bottom at the assembly point 6. This configuration ensures that small ticks that are not seized by the engagement faces 13 are not merely squeezed in the engagement part 2, but are rather conveyed into this slot and are subsequently pushed by or cut off by the bottom side 12 of the engagement part 2 at the bottom of the V-shaped indentation 4.

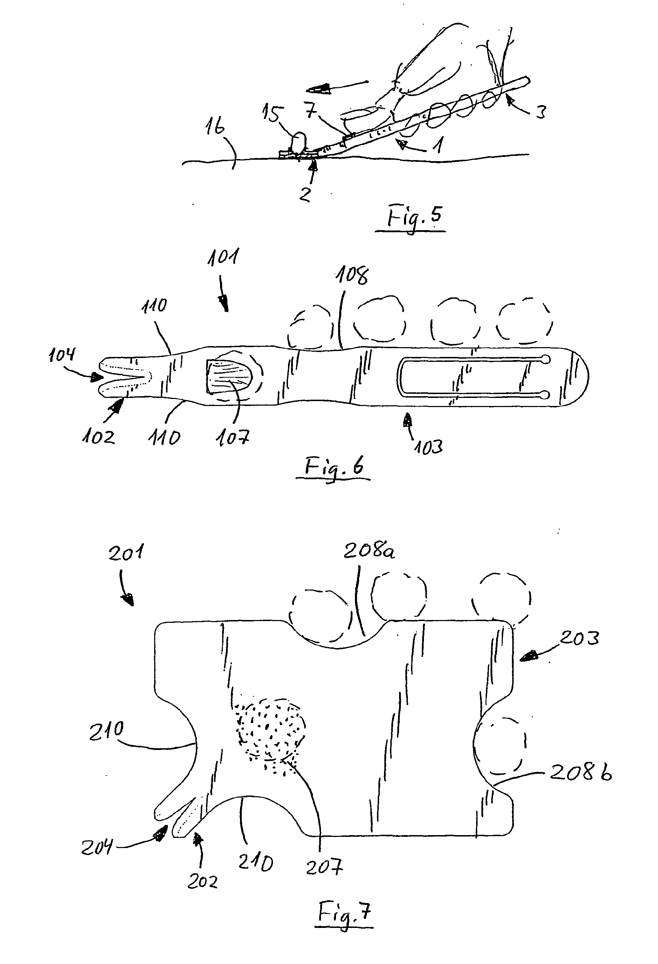

In the following the use of the tool1 will be described more closely, reference being made to Figure 4 that shows a cross-section through the engagement part 2 during removal of a tick 15 from the skin 16 of a person; reference is also made to Figure 5 that shows how the tool 1 is held during removal of a tick 15.

Firstly the position of the tick 15 on the skin is located, and as much hair or pelt as possible is pushed aside without the tick 15 being touched. Then the tool 1 is seized by the hand, the thumb being caused to abut on the upwardly protruding transverse beam 7 and the index finger being caused to abut in the recess 8. The engagement part 2 of the tool 1 now presses down towards the skin 16 with the opening of the V-shaped indentation pointing towards the tick 15. Owing to the auxiliary recesses 10, the tool 1 flexes in the transition between the engagement part 2 and the holder part 3, as shown in Figure 5.

The engagement part 2 of the tool 1 is now displaced in a swift movement towards and past the tick 15, whereby the latter is removed-the transition between the engagement faces 13 an the lowermost parts 11 a of the side faces 11 engaging the head 17 of the tick 15 and lifting the tick 15 off the skin 16, while the tool is being displaced across the skin. This is shown in Figure 4 which illustrates; by a fully drawn line, the initial position of the engagement part 2 in relation to a tick 15 in a situation where no contact has yet been established between the head 17 of the tick 15 and the transition between the engagement faces 13 and the lowermost parts 11 a of the side faces 11. When - according to the invention - the engagement part 2 is displaced in the direction of the arrow A (conf. Figure 2) across the skin 16, the transition between the engagement faces 13 and the lowermost parts 11 a of the side faces 11 engage the head 17 of the tick, and as the distance between the engagement face 13 and the bottom face of the engagement part 2 is increased towards the assembly point 6, the tick 15 will be lifted off the skin 16 as outlined by the dotted ticks.

In use the tool 1 is conveyed across the skin 16 in a swift, uninterrupted movement, and the elevation of the tick 15 therefore takes place within fractions of a second. Conversely to the known tools for removing ticks, use of the tool 1 having engagement faces 13 that move in a direction away from the bottom face 12, means that the tick 15 is lifted off the skin 16. This means that the tick is not manipulated in an adverse manner and thus the risk of it transmitting pathogenic bacteria is minimised.

In order to ensure that the inventive tool can be used efficiently, it is preferably provided with anergonomically correctly configured holder part. This may be obtained by configuring the holder part 3 as shown in Figure 1, where a transverse beam 7 and a recess 8 serve as stop for the user's thumb and index finger as described above.

An alternative embodiment of a tool according to the invention is shown in Figure 6 in the form of a pen-shaped tool 101. This tool 101 is plate-shaped throughout its entire length like the tool 1 shown in Figure 1 and it is further provided with a corresponding V-shaped indentation 104. The holder part 103 is provided with an indentation 107 for the thumb and with a recess 108 for the index finger. The positioning of the fingers is outlined by dotted lines in Figure 5. This embodiment, too, is provided with auxiliary recesses 110 in the transition between the engagement part 102 and the holder part 103 to increase the flexibility in this area.

According to a particular embodiment of a pen-shaped tool, the holder part may have a generally round or oval cross-section, while the engagement part is preferably still plate-shaped. This embodiment, however, is not shown.

An alternative embodiment of the inventive tool is shown in Figure 7 that shows a plate-shaped tool 201 having an outer contour of the same size as a credit card. The tool 201 is preferably manufactured from plastics having the same thickness as a credit card, but it may also be manufactured from metal. The tool 201 comprises an engagement part 202 in the one corner, said engagement part 202 being provided with a V-shaped indentation 204.

This tool, too, is provided with recesses 210 that enable the engagement part 202 to be flexed relatively easily in relation to the holder part 203 of the tool 201. The holder part 203 is moreover provided with a recess 208a for the index finger of the user and with a recess 208b for the user's little finger. Finally a ribbed area 207 is provided for the thumb of the user. The user's fingers are outlined by dotted lines, and the provision of the recesses 208a, 208b and the ribbed area 208 enables the user to obtain a safe grip around the tool 201 during use.

Advantageously this embodiment can be provided with two engagement parts 202 arranged in two neighbouring corners; thus it is obtained that the tool can be used in the same manner with both one's right and one's left hand.

As will appear from the above and from Figures 1,4, 5 and 6 it is of interest to provide the tool 1,101, 201 with means that ensure an ergonomically safe grip around the tool during use. This is to be seen in the context of the tool 1, 101,201 often being used in circumstances where the user has wet or slippery fingers. If the tool has rather smooth outsides without particular recesses or other means that may act as stops for one or more fingers, there is a risk of the tool slipping in the user's hand and thus it does not remove the tick effectively.

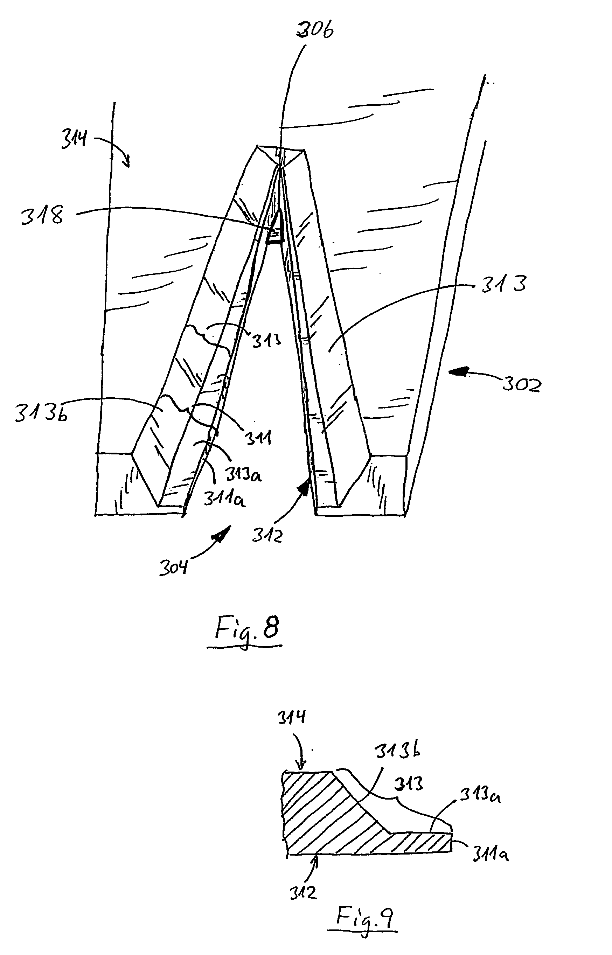

Figure 8 shows an alternative embodiment of the engagement part 302 for a tool according to the invention. In this embodiment the engagement part 302 is provided with engagement faces 313 that comprise a first part 313a which is in parallel with the bottom face 312 of the engagement part 302 and a second part 313b that extends as a plane face from the first part 313a to the top face 314 of the engagement part 302. By this configuration, the engagement face 313 becomes concave as will appear most clearly from Figure 9, which shows a cross-sectional view of the engagement face 313 in the engagement part 302 shown in Figure 8. By the concave configuration of the engagement faces 313, a space is formed between the engagement faces 313, whereby a tick can be removed without its head and/or body being influenced laterally as described above.

Also in this embodiment the transition between the engagement faces 313 and the lowermost part 311a of the side faces 311 diverge in a direction towards the assembly point 306 in the V-shaped indentation 304 from the bottom side 312 of the engagement part 302. The functioning of the engagement part 302 corresponds here in principle to the functioning of the engagement part 2 shown in Figures 2-4, ie the head of the tick is lifted upwards by the engagement faces 313 when the V-shaped indentation 304 is displaced in relation to the tick.

In the embodiment shown in Figure 8, the engagement part 302 is, at the bottom of the V-shaped indentation 304, provided with a cutter blade 318 that may consist of metal or other suitable material. The functioning of the cutter blade 318 is to cut off the head of the tick if, contrary to expectations, it is not accomplished to lift the tick off the skin. This may occur if the tick is extremely firmly embedded or if it is so small that the engagement faces 313 are unable to impart a sufficient lift to the tick.

If the tool for removing ticks is manufactured from an injection moulded plastics material, the cutter blade 318 may be constituted by an integrally moulded knife's blade or razor blade, or the cutter blade 318 may be constituted by an integral plastics part which is moulded with a very sharp front edge. Such a cutter blade may of course also be provided at the bottom of the V-shaped indentation in the embodiments shown in Figures 1-7.

The invention is described with reference to various embodiments of both the engagement part as such and the overall configuration of the tool. However, both of these elements can be varied beyond the disclosures of figures 1-9. The tool may preferably be configured as a relatively flat object as shown in Figures 1-9, whereby flexibility around the engagement part can easily be provided and thus an adequate and close contact with the skin during use is obtained. Moreover, the plate-shaped configuration means that the tool can easily be stored in various ways, eg in a pocket or along with the user's credit cards. However, nothing prevents the tool from being configured as a thicker unit, as long as care is being taken to ensure that the engagement faces are configured to be concave, thereby forming a "hollow" zone for receiving the head and/or body of the tick when the engagement part is displaced across said tick.

The tool is preferably manufactured by injection moulding of a plastics material that can be transparent or coloured. Alternatively the tool can be made of glass.

## Claims

1. A tool (1; 101; 201) for removing ticks (15) and other like parasites from the skin (16), which tool has an engagement part (2; 102; 202; 302) with a bottom face (12; 312) and a top face (14; 314), wherein a V-shaped indentation (4; 104; 204; 304) is provided that has side faces (11; 311) between the bottom face and the top face at an edge (5) of the engagement part (2; 102; 202; 302), which side faces (11; 311) converge towards each other from an outer opening at the edge (5) towards an internal assembly point (6; 306), said V-shaped indentation (4; 104; 204; 304) being wider at the top face (14; 314) than at the bottom face (12; 312), a considerable portion of each side face (11; 311) of the V-shaped indentation (4; 104; 204; 304) between the bottom face (12; 312) and the top face (14; 314) of the engagement part (2; 102; 202; 302) being constituted by a concave engagement face (13; 313), the lowermost part of the concave engagement face (13; 313) being substantially parallel with the bottom face (12; 312), the concave shape of the concave engagement face (13) being formed by a continuously curved line segment, **characterised in that**, at least for a distance, the lowermost part (11 a, 11 b; 311 a) of the side face (11; 311) is increasing in height, seen from the edge (5) to the internal assembly point (6; 306).

2. A tool as claimed in claim 1, **characterised in that** the concave shape of the concave engagement face (13) is formed by drawing of a circle.

3. A tool as claimed in claims 1 or 2, **characterised in that** the concave shape of the concave engagement face (13) is formed by a number of straight lines:

4. A tool as claimed in any one of claims 1-3, **characterised in that** each side face (11; 311) comprises a lowermost part (11 a, 11 b;311 a) that extends essentially perpendicularly from the bottom face (12; 312) and is connected to the concave engagement face (13; 313).

5. A tool as claimed in any one of claims 1 or 2, **characterised in that** an innermost part of the lowermost part(11b) of the side face (11) is provided with parallel side faces.

6. A tool as claimed in any one of claims 1-5, **characterised in that**, at the bottom of the V-shaped indentation (304) a cutter blade (318) is provided in level with the bottom face (312).

7. A tool as claimed in any one of claims 1-6, **characterised in that** it comprises a holder part (3) provided with an upwardly protruding transverse beam (7) for supporting a thumb.

8. A tool as claimed in any one of claims 1-7, **characterised in that** it comprises a holder part (103) provided with an indentation (107) for supporting a thumb.

9. A tool as claimed in any one of claims 1-8, **characterised in that** it comprises a holder part (203) provided with a ribbed area (207) for supporting a thumb.

10. A tool as claimed in any one of claims 1-9, **characterised in that** it comprises at least one recess (8_{;} 108; 208a, 208b) at the edge in support of at least one finger.

11. A tool as claimed in any one of claims 1-10, **characterised in** being plate-shaped.

12. A tool as claimed in claim 11, **characterised in that** at least one auxiliary recess (10; 110; 210) is provided in the area between the engagement part (2; 102; 202) and the holder part (3; 103; 203).

13. A tool as claimed in claims 11 or 12, **characterised in that** the largest outer dimension of the tool substantially corresponds to the size of a credit card.

## Patentansprüche

1. Werkzeug (1; 101; 201) zum Entfernen von Zecken (15) und anderen, ähnlichen Parasiten von der Haut (16), welches Werkzeug ein Eingriffstell (2; 102; 202; 302) mit einer Unterseitenfläche (12; 312) und einer Oberseitenfläche (14; 314) aufweist, wobei eine V-förmige Einbuchtung (4; 104; 204; 304) bereitgestellt ist, welche Seitenflächen (11; 311) zwischen der Unterseitenfläche und der Oberseitenfläche an einem Rand (5) des Eingriffstells (2; 102; 202; 302) aufweist, welche Seitenflächen (11; 311) aufeinander zu, von einer äußeren Öffnung an dem Rand (5) in Richtung auf einen internen Verbindungspunkt (6; 306) zu konvergieren; wobei die V-förmige Einbuchtung (4; 104; 204; 304) an der Oberseitenfläche (14; 314) breiter als an der Unterseitenfläche (12; 312) ist, wobei ein beachtlicher Abschnitt von Jeder Seitenfläche (11; 311) der V-förmigen Einbuchtung (4; 104; 204; 304) zwischen der Unterseltenfläche (12; 312) und der Oberseitenfläche (14; 314) des Eingriffsteils (2; 102; 202; 302) von einer konkaven Eingriffsfläche (13; 313) gebildet ist, wobei der unterste Teil der konkaven Eingriffsfläche (13; 313) im Wesentlichen parallel zu der Unterseitenfläche (12; 312) ist, wobei die konkave Form der konkaven Eingriffsfläche (13) von einem kontinulerlich gekrümmten Liniensegement gebildet ist, **dadurch gekennzeichnet, dass**, zumindest für eine Strecke, der : unterste Teil (11a, 11b; 311a) der Seitenfläche (11; 311) gesehen von dem Rand (5) zu dem internen Verbindungspunkt (6; 306) an Höhe zunimmt.

2. Werkzeug nach Anspruch 1, **dadurch gekennzeichnet, dass** die konkave Form der konkaven Eingriffsfläche (13) durch Ziehen eines Kreises gebildet ist.

3. Werkzeug nach Anspruch 1 oder 2, **dadurch gekennzeichnet, dass** die konkave Form der konkaven Eingriffsfläche (13) durch eine Anzahl von geraden Linien gebildet ist.

4. Werkzeug nach einem der Ansprüche 1 bis 3, **dadurch gekennzeichnet,**
**dass** jede Seltenfläche (11; 311) einen untersten Teil (11a, 11b; 311a) umfasst, welcher sich im Wesentlichen senkrecht von der Unterseitenfläche (12; 312) erstreckt und mit der konkaven Eingriffsfläche (13; 313) verbunden ist.

5. Werkzeug nach einem der Ansprüche 1 oder 2, **dadurch gekennzeichnet,**
**dass** ein innerster Teil des untersten Teils (11b) der Seitenfläche (11) mit parallelen Seitenfläche bereitgestellt Ist.

6. Werkzeug nach einem der Ansprüche 1 bis 5, **dadurch gekennzeichnet,**
**dass** an der Unterseite der V-förmigen Einbuchtung (304) ein Schneldblatt (318) auf Niveau der Unterseitenfläche (312) bereitgestellt ist.

7. Werkzeug nach einem der Ansprüche 1 bis 6, **dadurch gekennzeichnet, dass** es ein Haltertell (3) umfasst, welches mit einem nach oben vorstehenden Querbalken (7) zum Unterstützen eines Daumens bereitgestellt ist.

8. Werkzeug nach einem der Ansprüche 1 bis 7, **dadurch gekennzeichnet,**
**dass** es ein Halterteil (103) umfasst, welches mit einer Einbuchtung (107) zum Unterstützen eines Daumens bereitgestellt ist.

9. Werkzeug nach einem der Ansprüche 1 bis 8, **dadurch gekennzeichnet, dass** es ein Halterteil (203) umfasst, welches mit einem gerippten Bereich (207) zum Unterstützen eines Daumens bereitgestellt ist.

10. Werkzeug nach einem der Ansprüche 1 bis 9, **dadurch gekennzeichnet,**
**dass** es wenigstens eine Aussparung (8; 108; 208a, 208b) an dem Rand zur Unterstützung von wenigstens einem Finger umfasst.

11. Werkzeug nach einem der Ansprüche 1 bis 10, **dadurch gekennzeichnet, dass** es plattenförmig ist.

12. Werkzeug nach Anspruch 11, **dadurch gekennzeichnet, dass** wenigstens eine Hilfsaussparung (10; 110; 210) in dem Bereich zwischen dem Eingriffstell (2; 102; 202) und dem Halterteil (3; 103; 203) bereitgestellt ist.

13. Werkzeug nach Anspruch 11 oder 12, **dadurch gekennzeichnet, dass** die größte äußere Abmessung des Werkzeugs im Wesentlichen der Größe einer Kreditkarte entspricht.

## Revendications

1. Instrument (1 ; 101 ; 201) pour l'élimination de tiques (15) et autres parasites similaires de la peau (16), lequel instrument possède une partie de mise en prise (2 ; 102 ; 202 ; 302) avec une face inférieure (12 ; 312) et une face supérieure (14; 314), dans lequel est prévue une indentation en V (4 ; 104 ; 204 ; 304) qui présente des faces latérales (11 ; 311) entre la face inférieure et la face supérieure sur un bord (5) de la partie de mise en prise (2 ; 102 ; 202 ; 302), lesquelles faces latérales (11 ; 311) convergent l'une vers l'autre depuis une ouverture externe sur le bord (5) vers un point d'assemblage interne (6 ; 306), ladite indentation en V (4 ; 104 ; 204 ; 304) étant plus large au niveau de la face supérieure (14 ; 314) qu'au niveau de la face inférieure (12 ; 312), une partie considérable de chaque face latérale (11 ; 311) de l'indentation en V (4 ; 104 ; 204 ; 304) entre la face inférieure (12 ; 312) et la face supérieure (14; 314) de la partie de mise en prise (2 ; 102; 202 ; 302) étant constituée par une face de mise en prise concave (13 ; 313), la partie la plus basse de la face de mise en prise concave (13 ; 313) étant sensiblement parallèle à la face inférieure (12 ; 312), la forme concave de la face de mise en prise concave (13) étant formée par un segment linéaire incurvé en continu, **caractérisé en ce que**, au moins sur une certaine distance, la partie la plus basse (11a, 11b ; 311a) de la face latérale (11 ; 311) a une hauteur croissante, vue depuis le bord (5) vers le point d'assemblage interne (6; 306).

2. Instrument selon la revendication 1, **caractérisé en ce que** la forme concave de la face de mise en prise concave (13) est formée en traçant un cercle.

3. Instrument selon la revendication 1 ou 2, **caractérisé en ce que** la forme concave de la face de mise en prise concave (13) est formée par un certain nombre de lignes droites.

4. Instrument selon l'une quelconque des revendications 1 à 3, **caractérisé en ce que** chaque face latérale (11 ; 311) comprend une partie la plus basse (11a, 11b; 311a) qui s'étend de façon essentiellement perpendiculaire à partir de la face inférieure (12 ; 312) et est connectée à la face de mise en prise concave (13 ; 313).

5. Instrument selon l'une quelconque des revendications 1 ou 2, **caractérisé en ce qu'**une partie la plus interne de la partie la plus basse (11b) de la face latérale (11) est dotée de faces latérales parallèles.

6. Instrument selon l'une quelconque des revendications 1 à 5, **caractérisé en ce que**, au fond de l'indentation en V (304), est prévue une lame de découpe (318) de niveau avec la face inférieure (312).

7. Instrument selon l'une quelconque des revendications 1 à 6, **caractérisé en ce qu'**il comprend une partie de maintien (3) dotée d'une poutre transversale faisant saillie vers le haut (7) pour supporter un pouce.

8. Instrument selon l'une quelconque des revendications 1 à 7, **caractérisé en ce qu'**il comprend une partie de maintien (103) dotée d'une indentation (107) pour supporter un pouce.

9. Instrument selon l'une quelconque des revendications 1 à 8, **caractérisé en ce qu'**il comprend une partie de maintien (203) dotée d'une zone nervurée (207) pour supporter un pouce.

10. Instrument selon l'une quelconque des revendications 1 à 9, **caractérisé en ce qu'**il comprend au moins un évidement (8 ; 108 ; 208a, 208b) sur le bord pour supporter au moins un doigt.

11. Instrument selon l'une quelconque des revendications 1 à 10, **caractérisé en ce qu'**il est de forme plate.

12. Instrument selon la revendication 11, **caractérisé en ce que** l'au moins un évidement auxiliaire (10 ; 110 ; 210) est prévu dans la zone entre la partie de mise en prise (2 ; 102 ; 202) et la partie de maintien (3 ; 103 ; 203).

13. Outil selon la revendication 11 ou 12, **caractérisé en ce que** la dimension externe la plus large de l'outil correspond sensiblement à la taille d'une carte de crédit.
